# EUROPEAN PATENT SPECIFICATION

(11) **EP 1 891 436 B1**
(45) Date of publication and mention of the grant of the patent: **11.07.2012**
(21) Application number: 06742438.2
(22) Date of filing: 29.05.2006
(51) Int. Cl.: G01N 33/50, C12Q 1/06

(54) **QUANTIFICATION OF THE VIABILITY OF LACTIC ACID BACTERIA USING FLOW CYTOMETRY**
QUANTIFIZIERUNG DER LEBENSFÄHIGKEIT VON MILCHSÄUREBAKTERIEN UNTER VERWENDUNG VON DURCHFLUSSZYTOMETRIE
QUANTIFICATION DE LA VIABILITE DES BACTERIES DE L'ACIDE LACTIQUE PAR CYTOMETRIE DE FLUX

(30) Priority: 27.05.2005 DK 200500777
(43) Date of publication of application: 27.02.2008
(60) Divisional application: 12150893.1
(73) Proprietor: Chr. Hansen A/S, 2970 Hørsholm (DK)
(72) Inventor: WORM, Jacob, DK-2720 Vanløse (DK); STAVNSBJERG, Rikke, DK-2860 Søborg (DK); MØLLGAARD, Henrik, DK-2800 Lyngby (DK)
(86) International application number: PCT/DK2006/000298
(87) International publication number: WO 2006/125446

(56) References cited:
- DATABASE MEDLINE [Online] US NATIONAL LIBRARY OF MEDICINE (NLM), BETHESDA, MD, US; May 2001 (2001-05), BUNTHOF C J ET AL: "Flow cytometric assessment of viability of lactic acid bacteria." XP002404636 Database accession no. NLM11319119 & APPLIED AND ENVIRONMENTAL MICROBIOLOGY. MAY 2001, vol. 67, no. 5, May 2001 (2001-05), pages 2326-2335, ISSN: 0099-2240
- DATABASE MEDLINE [Online] US NATIONAL LIBRARY OF MEDICINE (NLM), BETHESDA, MD, US; November 2002 (2002-11), AMOR KAOUTHER BEN ET AL: "Multiparametric flow cytometry and cell sorting for the assessment of viable, injured, and dead bifidobacterium cells during bile salt stress." XP002404637 Database accession no. NLM12406706 & APPLIED AND ENVIRONMENTAL MICROBIOLOGY. NOV 2002, vol. 68, no. 11, November 2002 (2002-11), pages 5209-5216, ISSN: 0099-2240

## Description

### FIELD OF INVENTION

The present invention pertains to a method of quantifying the number of viable lactic acid bacteria (LAB) cells in a sample.

### BACKGROUND OF INVENTION

Bacteria which ferment sugars with the production of acids In particular lactic acid as a major metabolic component has been known for a long time. Such bacteria may be found in milk or milk products, living or decaying plants but also in the intestine of man and animals. Traditionally, these bacteria has been referred to as "lactic acid bacteria". Lactic acid bacteria (LAB) designates a rather heterologous group of Gram positive, non-motile, microaerophilic or anaerobic bacteria which ferment sugar with the production of acids including lactic acid and comprise e.g. the genera *Bifidobacterium, Enterococcus, Lactobacillus, Lactococcus, Leuconostoc* and *Pediococcus.* For centuries starter cultures of lactic acid bacteria have been used in the manufacture of food and feed products including most dairy products and today lactic acid bacteria are essential in the making of all fermented milk products such as yoghurt, cheese and butter. Furthermore lactic acid bacteria are widely used in the meat processing industry, wine manufacturing industry, the juice manufacturing industry as well as a number of other industries. The publication of a large amount of reports documenting that various lactic bacteria beneficially affect the well-being of humans and/or animals have attracted even further interest to this group of bacteria. In particular, specific strains of Lactobacillus or *Bifidobacterium* have been found to be able to colonize the intestinal mucosa and to assist in the maintenance of the well-being of the hosts. Live microorganisms which when administered in adequate amounts confer a health benefit on the host, is referred to as probiotic organisms or probiotics (FAO / WHO 2002).

Both the use as starter culture and as probiotics imply that the LAB are exposed to various stress conditions that may affect their viability. Both starter cultures and probiotics are for practical reasons often provided in a freeze dried form. It is well known in the art that the number of viable cells of lactic acid bacteria is significantly reduced during the process of freeze drying. As the metabolic activities that characterizes live cells are essential both for the use as starter cultures and as probiotics, the content of live cells should be established for each batch of a starter culture or a probiotic composition. Furthermore, it also well known that the number of live cells decreases during storage. Thus, to ensure that the products provide sufficient live cells the shelf life of the products should be checked by monitoring the amount of live cells in spot tests at regular intervals.

Conventionally, the contents of viable cells have been performed by the determination of colony forming units (CFU) by the plate count technique, in which the formation of visible colonies on an appropriate agar growth medium is followed.

Living cells are characterized by an electrical potential gradient with the cytosol being electrical negative with respect to the exterior environment. Maintenance of the membrane potential requires an active energy metabolism in eukaryotic as well as in bacteria cells. Since the expenditure of metabolic energy is required to maintain potentials, the potential across the membrane of an injured or dying cell is decreased in magnitude. In bacteria the membrane potential is decreased or lost within minutes following removal of energy sources. Membrane potential is also decreased or lost if the bacterial cell membrane is ruptured or otherwise damaged by physical or chemical means (Shapiro, (1990) ASM news 56, 584-588). Thus, the level of the membrane potential is a highly sensitive indicator not only of the membrane integrity but of the general physiological state of the individual bacteria cell.

Novo et al. (1999) describe an assay to determine the membrane potential for single bacterial cells. They base their assay on the fluorescent dye 3,3'-diethyloxacarbocyanine iodide (DiOC2(3); DiOC). DiOC is lipophilic, but also positively charged. The dye stains bacteria with green fluorescence, which presumably reflects the distribution of dye toward lipids in the membrane and hydrophobic constellations of the cell. Cellular energy generation leads to an electrochemical gradient across the membrane. The electrical potential (inside negative) changes the distribution of DiOC via an inward electroforesis. As discussed by Novo et al. (1999) the accumulation of dye probably leads to the formation of dye aggregates in the aqueous parts of the cytosol which result in a red-shift in the fluorescence emission of the dye which is indicative of cells with a "normal" membrane potential.

Flow cytometry (FCM) is a rapid technique for single cell analysis. In FCM individual cells are analysed at rates of 100 to 1000 per s as they are carried within a fast-flowing fluid that passes a laser light source typically the forward angle light scatter (FSC), the side-angle light scatter (SSC), and the fluorescence of individual cells at selected wavelengths are measured.

A number of different fluorescent probes have been developed for viability assessment by use of FCM-techniques. Literature provide examples of the use of fluorescent probes to monitor various physiological parameters such as DNA content, enzyme activity, respiration, membrane potential, intracellular pH, and membrane integrity at the single cell level (Bunthof, 2001 and Ben Amor, 2002). In particularly, viability assays that are based on detecting membrane integrity have enjoyed wide use. Recently, however, it was reported that CFU determinations do not appear to be related to results obtained with viability test based on cell membrane integrity during storage of *Bifidobacteria* (Lathinen, 2005). As reported in example 1 we have observed similar results with Lactobacillus cells during storage.

To conclude, the CFU determination is the standard reference method for viability testing used by the dairy industry. Unfortunately, the determination of colony forming units is a rather slow and very labour-intensive assay which it has proved very difficult to automate. Thus, there is a persisting need for methods that allows for a fast and reliable quantification of viable cells and which can be correlated to the number of CFU.

### SUMMARY OF INVENTION

The problem underlying the present invention was to provide a fast and reliable method of quantifying the amount of live LAB in a sample. It was desired that the method of quantifying the number of viable lactic acid bacteria (LAB) cells in a sample should be sufficient "resilient" to variations in assay times that is a characteristic of high throughput screening.

To obtain an efficient, high-throughput viability testing protocol we have invented an FCM-based method of quantifying the viability of LAB that easily can be linked up to a liquid handling robot and thus automated. However, to obtain a satisfactory throughput the various steps in the procedure must be performed sequentially, the result being that the process time of an individual sample approaches one hour, during which the number of viable cells in a sample unfortunately is not constant.

The present invention is based on the surprising finding that the cellular membrane potential of LAB cells is very sensitive to the actual conditions under which the cells are stained and kept until the actual FCM analysis is performed, and this may be the reason why the known methods did not work properly. As shown in Examples 2 and 3 the composition of the actual staining mix is of major importance to the resiliency of the assay. The "resilience" or the "resiliency" of the analytical method refers to the degree of reproducibility of test results obtained by the analysis of the same (or similar) samples under a variety of normal test conditions, such as in different laboratories, different analysts, different instruments, different lots of reagents, different elapsed assay times, different assay temperatures, different days, etc.

Thus, it has surprisingly turned out that when keeping the cells under conditions where the cell membrane potential is kept substantially constant during the FCM quantification, it is possible to provide a method for high-throughput viability testing. It is, however, envisaged that the novel invention can be used in other viability testing methods wherein a reliable and fast testing is desired.

In accordance herewith, the invention provides to a method of quantifying the number of viable lactic acid bacteria (LAB) cells in a sample which is sufficient "resilient" to variations in assay times that is a characteristic of automation according to claim 1. The method comprise the steps of:
(1) mixing said LAB cells with a dye, which associates with individual LAB cells and which is indicative of the cell membrane potential of said individual cells and a complex growth medium in a concentration that in 2% to 10% of the recommended concentration when used as a growth medium for LABs ;
(2) incubating the cells under conditions where viable cells retain said cell membrane potential while their growth is inhibited,
(3) detecting an optical property of the dye composition associated with said individual cells; and
(4) quantifying the number of viable cells in the sample.

### DETAILED DISCLOSURE

In a first aspect, the present invention relates to a method of quantifying the number of viable lactic acid bacteria (LAB) cells in a sample according to claim 1 comprising the steps of:
1: mixing said LAB cells with a dye, which associates with individual LAB cells and which is indicative of the cell membrane potential of said individual cells and a complex growth medium in a concentration that in 2% to 10% of the recommended concentration when used as a growth medium for LABs ;
2: incubating under conditions where viable cells retain said cell membrane potential while their growth is inhibited ;
3: detecting an optical property of the dye composition associated with said individual cells; and
4: quantifying the number of viable cells in the sample.

The optical property of the dye composition may be detected using any suitable means known in the art. A microfluorometer may be used to measure fluorescence, but the measurement is accomplished most rapidly and precisely using a flow cytometer. The methodology of flow cytometry and cell sorting is described by P.K. Horan and L.L. Wheeless, Jr. in Science, Vol. 198, pages 149-157 (1977). Flow cytometers employing argon ion laser light sources are well suited for the present measurements. Such instruments are sold by several manufactureres, and are capable of automatically sorting cells on the basis of several criteria, e.g., fluorescent intensity, cell size, wavelength of fluorescence, and wavelength or intensity of absorption.

The dye composition that is mixed with LAB cells in step (1) may comprise any suitable dye known in the art. Additionally, it has been found by the present inventor that it is highly advantageous if the dye composition further comprises a diluted complex growth media. Complex growth media are well known to the person of skill in the art and a suitable media is MRS (de Man et al. 1960). The purpose of adding a diluted growth media to the dye composition is to maintain (retain) a membrane potential in the cells without any significant proliferation of the cells i.e. active growth of the cells is inhibited and to obtain a degree of resilience of the cells as described above. Obviously, the degree of dilution depends of the type of LAB, the media, incubation conditions etc. Typically, the growth media will be diluted to a final concentration in the range of 2-10% (vol/vol), including a range of 2-5% (vol/vol). Interestingly, the examples herein shown that a dilution below 1% (vol/vol) does not sustain a stable membrane potential. Thus, a suitable dilution of the growth medium is a dilution to a final concentration of at least 2%, such a dilution to a final concentration of 2%, 2,5%, 3%, 3,5%, 4%, 4,5%, 5% or even 6% (vol/vol).

It is envisaged that the diluted growth medium and the dye may be mixed/contacted with the cells in either one step or in two steps. Accordingly, the application relates to a method of quantifying the number of viable lactic acid bacteria (LAB) cells in a sample comprising the steps of:
1: contacting said LAB cells (or the sample) with i) a dye which associates with individual LAB cells and which is indicative of the cell membrane potential of said individual cells, and ii) and a composition which makes viable cells retain said cell membrane potential while their growth is inhibited, and/or
   contacting said LAB cells (or the sample) with a dye composition which makes viable cells retain said cell membrane potential while their growth is inhibited, said composition comprising a dye which associates with individual LAB cells and which is indicative of the cell membrane potential of said individual cells;
2: incubating the cells;
3: detecting an optical property of the dye associated with said Individual cells; and
4: quantifying the number of viable cells.

It may be beneficial to add a carbohydrate such as e.g. glucose to the dye composition comprising diluted growth medium. The carbohydrate may be added in a concentration which is in the range of 0,5-10%, such as in the range of 1 - 10% including a range of 2-5% vol/vol (final concentration).

A further condition that influences the resilience of the cells as defined herein is the temperature during the mixing and incubation steps. The applied temperature may be selected with respect to the optimal growth temperature of the specific LAB cells. Typically, the applied temperature is lower than the temperature optimal for growth. A suitable temperature is at least 10°C lower than the optimal growth temperature of the lactic acid bacteria cells.

In the present context, the expression "lactic acid bacteria" designates a group of Gram positive, catalase negative, non-motile, microaerophilic or anaerobic bacteria which ferment sugar with the production of acids including lactic acid as the predominantly produced acid, acetic acid, formic acid and propionic acid. The industrially most useful lactic acid bacteria are found among *Lactococcus* species, *Streptococcus* species, *Enterococcus* species, *Lactobacillus* species, *Leuconostoc* species, *Pediococcus* species and *Bifidobacterium* species.

Commonly, strains of lactic acid bacteria are generally divided into mesophilic organisms having optimum growth temperatures at about 30°C and thermophilic organisms having optimum growth temperatures in the range of about 40 to about 45°C. Typical organisms belonging to the mesophilic group include *Lactococcus lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis, Lactobacillus casei* subsp. *casei* and *Lactobacillus paracasei* subsp. *paracasei.* Thermophilic lactic acid bacterial species include as examples *Streptococcus thermophilus, Enterococcus faecium, Lactobacillus delbrueckii* subsp. *lactis, Lactobacillus helveticus, Lactobacillus delbrueckii* subsp. *bulgaricus* and *Lactobacillus acidophilus.*

Thus, optimal growth temperatures for lactic acid bacterial are found within a broad temperature range such as in a range from 15°C to 50 °C. A suitable temperature for use in the present method is selected in the range between 0°C to 40°C, such as in a range of 10 °C to 25°C, including a range between 15 °C to 20°C. As illustrated in the examples herein the temperature applied in the present method is a temperature of 20-21 °C when the organism is a thermophilic LAB such as those used in the examples.

As previous mentioned it is an essential feature of the present method that the viable cells to be quantified retain (maintain) their cell membrane potential for a significant period of time, i.e. a period of time that allows for handling and optical detection of large numbers of samples. Typically, the membrane potential is maintained for at least 10 minutes, such as at least 20 minutes, such as at least 40 minutes, such as at least 50 minutes. The membrane potential may be retained for at least 1 hour, such as at least 1,5 hours, such as at least 2 hours or even for at least 3 hours.

The LAB to be quantified with respect to viability according to the present method may be provided in any form known in the art. Commercial sold LAB cultures may be provided in a freeze dried form. When the LAB cells are freeze dried, or otherwise prepared for storage, it may be beneficial to apply a rehydration step and/or an activation step prior to the mixing step (1) as described above. Rehydration may be carried out in any suitable aqueous medium such as e.g. water, growth medium, a buffer solution etc. The cells may further be subjected to an activation step such an activation are typically performed by subjecting the cells to conditions preparing the cells to active growth. A suitable activation comprise incubation the cells in a complex growth medium. If the activation step comprises incubation in a complex growth medium, it is clear to the skilled person that before quantification the sample containing the LAB should either be diluted in order to keep the concentration of the growth medium component so low that bacterial growth is not sustained, or the LAB cells should be isolated e.g. by centrifugation or filtration.

Suitable dyes for use in the present method include but are not limited to a "symmetric" cyanine dye and a dye selected from the group of dyes with the formulas:
a) 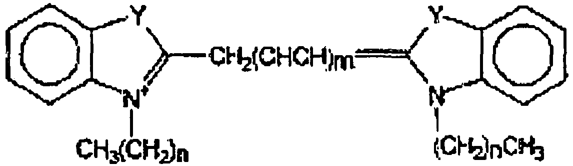 wherein Y designates O, S, or a group C(X)Z (wherein X and Z independently designate hydrogen or C1-C10 alkyl); m and n independently designate an integer between 0 and 10,
and b)

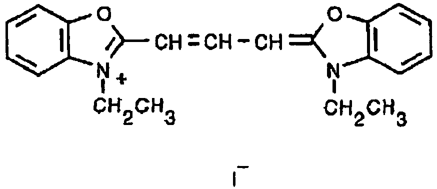

Further disclosed is a composition usable for staining of lactic bacteria cells, said compostion comprises:
a) a dye; and
b) components which make viable cells retain (or substantially retain) their cell membrane potential while their growth is inhibited (or substantially inhibited).

It is presently preferred that the dye associates with the bacteria cell, thereby being indicative of the cell membrane potential. The composition may comprise bacteria cells, examples on such compositions are:

A composition comprising:
a) LAB cells;
b) a dye which associates with individual LAB cells and which is indicative of the cell membrane potential of said individual cells; and
c) components which make viable cells retain (or substantially retain) said cell membrane potential while their growth is inhibited (or substantially inhibited).

A composition comprising:
a) LAB cells;
b) a dye which associates with individual LAB cells and which is indicative of the cell membrane potential of said individual cells; and
c) components which make viable cells retain (or substantially retain) said cell membrane potential while their growth is inhibited (or substantially inhibited), said components being a yeast extract, a beef extract, a peptone, a tryptone or the like, such as in a concentration of 0.05% to 0.25% (dry matter), and/or said components being the components of a medium usable for the growth of LAB cells (such as MRS or MRS-CM), but in a concentration 2% to 10% of the concentration compared to when the components are used for preparation of a growth medium.

The dye may be a dye as defined above, or a dye selected from the group consisting of: a dye as defined in claim 12; a dye as defined in claim 13; a dye as defined by Sims et al (1974); a dye as defined by Novo et al (1999).

The composition comprises components that are able to sustain cell viability, but are not able to sustain cell growth. Thus, the composition may contain components belonging to the group consisting of:
a) growth media containing yeast extract; beef extract; tryptone; MRS-broth; and/or peptone;
b) MRS-broth; or MRS-CM.

When the composition comprises bacteria cells, such cells are preferable selected from the cells defined above.

Further, the application relates to a composition which is obtainable by a method comprising the following steps:
1: diluting a medium usable for growth of LAB cells 10 to 50 times, e.g. with a buffer or water;
2: adding dye to the diluted medium; and
3: adding LAB cells to the diluted medium.

Examples of growth media, dyes and LAB cells are described above. The composition may contain further ingredients, such as a carbohydrate or a mineral salt.

Further disclosed is an apparatus consisting of a detector (such as a flow cytometry instrument, e.g. detecting fluorescence) operatively connected to a container comprising a composition of the application.

An interesting embodiment of the invention relates to a method of quantifying the number of viable lactic acid bacteria (LAB) cells (e.g. in a sample) according claim 1 comprising the steps of:
1: preparing a composition comprising:
   a) the LAB cells;
   b) a dye which associates with individual LAB cells and which is indicative of the cell membrane potential of said individual cells;
   c) a complex growth medium in a concentration that in 2% to 10% of the recommended concentration when used as a growth medium for LABs ,
2: incubating the cells;
3: detecting an optical property of the dye associated with said individual cells; and
4: quantifying the number of viable cells.

By the term "components which makes viable cells retain (or substantially retain) said cell membrane potential while their growth is inhibited (or substantially inhibited)" should be understood components that are able to sustain cell viability, but not to sustain (significant) cell growth. The components in 1c) may be the components of a complex growth medium as defined herein, preferably in a concentration that is 2% to 10% (calculated of the composition as a whole) of the recommended concentration when used a growth medium for LABs. It means that the compostion may be based on e.g. a MRS growth medium diluted from 5 to 50 times, or 10 to 50, such as with a buffer (e.g. PBS) or water.

Other interesting embodiments of the method of the invention are:
- a method wherein the composition or dye composition in step (1) comprises a growth medium, such as a diluted and/or complex growth medium; especially wherein the growth medium contains yeast extract; beef extract; tryptone; peptone; or MRS-broth.
- a method wherein the dye composition comprises a growth medium at a concentration which is 2-10% vol/vol (final concentration), preferably 3-10, or
- a method wherein the incubation in step (2) is performed at a temperature, which is at least 10°C lower than the optimal growth temperature of the lactic acid bacteria cells, such as at about 10 °C; about 20°C; about 25 °C, or in the range 10°C to 25 °C;
- a method wherein the detection in step (3) is performed by flow cytometry, e.g. by measuring fluorescence;
- a method wherein the lactic acid bacteria cells are thermopilic lactic acid bacteria cells;
- a method wherein the viable cells retain their cell membrane potential for at least 1 hour;
- a method wherein the dye composition further comprises glucose at a concentration which is 0,5-10%, preferably 1 - 10% and most preferred 2-5% vol/vol (final concentration);
- a method wherein the sample of LAB cells is a composition that comprises freeze dried LAB cells;
- a method wherein the method further comprises the steps of:
   a) rehydration of said cells in an aqueous medium; and
   b) activation of said cells by incubation in a complex growth medium, prior to the mixing in step (1); and
- a method wherein the dye composition comprises a cyanine dye (preferably a "symmetric" cyanine dye), such as a lipolytic dye with a delocalisized positive charge, comprising two heterocyclic rings (preferably identical) joined by a polymethine bridge,
   e.g. a dye as described by Sims et al (1974) or Novo et al (1999). A specific dye may be DiOC as described herein, or DiOC2(3); DiOC1(3); DiOC4(3); DlIC5(3); DiIC6(5); DiSC3(5); DiBAC4(3); DiIC1(5); DiOC6(3); DiIC1(3); DiSC6(5) as defined by Novo et al (1999)

Having generally described the aspect and features of the present method, the invention will now be described using specific examples and figures. The figures and examples further illustrate various features and advantages of the invention, but are not intended to limit the scope of the invention.

### LEGENDS TO FIGURES

Figure 1. Comparison of number of viable cells of L. acidophilus strain LA-5 after accelerated storage in air tight aluminium bags for 0-3 weeks at 30°C as determined by a FCM membrane integrity assay and determination of colony forming units (CFU) by the plate count technique.
Figure 2. Number of viable cells of L. acidophilus strain LA-5 after accelerated storage in air tight aluminium bags for 3 weeks at 30°C (Control: -50°C). One standard deviation of the mean is inicated by upward bars (In-tact membrane counts) or downward bars (CFU).
Figure 3. The fraction of cells in a sampe of L. acidophilus strain LA-5 cells with a membrane potential that incates viability stained and kept in stain-mix's comprising 1, 4 or 10% MRS for 5 to 140 minutes.
Figure 4. Correspondance between counts of cells expressing membrane potential expressing cells (Active event/g product) versus the traditional CFU counts. The line indicates a 1:1 ration. The correlation coefficient (r²) is 0.79. Bars indicate ±1 standard deviation of replicate analyses.
Figure 5. Survival of *Lactobacillus acidophilus* strain LA-5 during test of accelerated storage stability. Bars indicate +1 standard deviation.
Figure 6. Eight samples analysed repeatedly with approx. 10 minutes intervals.
Figure 7. Linearity between amount of freeze-dried (FD) bacteria applied to the assay and counts of cells (events). Data are included for three independent experiments (Expt. 1 - 3; see legends). The regression coefficient is 0.996.

### DEFINITIONS

In the present context, the term "substantially retain" refers to that at least 80% (such as at least 90% or at least 95%) of the cell membrane potential is retained for a period of 60 minutes at 20 degrees C when the cells are incubated in the "stain-mix preparation" as defined herein, or it refers to that at least 80% (such as at least 90% or at least 95%) of the cells remain viable for a period of 60 minutes at 20 degrees C when the cells are incubated in the "stain-mix preparation" as defined herein.

The term "substantially inhibited" refers to that at most 20% (such as at most 10% or at most 5%) of the cells divide for a period of 60 minutes at 20 degrees C when the cells are incubated in the "stain-mix preparation" as defined herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value failing within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### EXAMPLES

### MATERIALS AND METHODS

### Bacteria and Production batches

Freeze-dried powders were used for the following human health strains: LA-5, BB-12 and CRL-431, see details in Table 2.2.1a. Note that two products of LA-5 are used. If not otherwise stated LA-5 refers to material no. 501084/Batch 2484524.

**Table 2.2.1a. Data on bacterial strains used in the present work.**

| Short name | Species | CHCC | Production data |
|---|---|---|---|
| LA-5 | *Lactobacillus acidophilus* | 3777 | Mat: 501084 / Batch: 2484524 |
| | | 2169 | Mat: 615939 / Batch: 2490471 |
| BB-12 | *Bifidobacterium animalis* subsp. *lactis* | 2186 | Mat: 615940 / Batch: 2480410 |
| CRL-431 | *Lactobacillus paracasei* subsp. *paracasei* | 3136 | Mat: 621008 / Batch: 2238008 |

### Media and reagents

### DiOC

DiOC is prepared eg. as a stock of 1.5 mM in DMSO:
1. An appropriate container is tared on a balance with mg resolution
2. DiOC (Note 1) powder is applied and the weight is recorded
3. Dissolve all powder in a volume of DMSO (Note 3) equivalent to 0.69 mg/ml final concentration (1.5 mM)
4. Aliquote, e.g. 1 ml to Eppendorf tubes
5. Store in darkness at ca. 5°C after appropriate labelling

**Note 1.** DiOC abbreviates 3,3'-diethyloxacarbocyanine iodide (often specified as DiOC₂(3)). Molecular formula: C₂₁H₂₁IN₂O₂; Molecular weight: 460.31; CAS Number/Name: 905-96-4 /3-ethyl-2-[3-(3-ethyl-2(3H)-benzoxazolylidene)-1-propenyl]-benzoxazolium iodide. DiOC is provided by Molecular Probes, Leiden, The Netherlands (Cat.no. D14730).

**Note 2.** DiOC absorbs light and is potentially unstable in light. However, no loss of staining capacity has been observed despite repeated freeze-thaw cycles and unwanted storage at room temperature.

**Note 3.** DMSO is dimethylsulfoxide (CAS Number: 67-68-5).

**Note 4.** Certain tubes of plastic react with DMSO giving a frosted appearance of the plastic. Do not use such plastics for the DiOC stock.

**Note 5.** DMSO is frozen at ca. 5°C and the DiOC stock kept at this temperature needs to thaw before use.

### 50% glucose

A stock solution of 50% glucose is prepared, e.g. as follows (exemplified for 100 ml):
1. 50 g D(+)-glucose-monohydrate (MW = 198.17)
2. Add 50 g demineralised water
3. Dissolve, e.g. aided by warming to below the boiling point
4. Aliquote, e.g. 1 ml to eppendorf tubes
5. Store frozen

### Bead-Mix preparation

Bead-Mix is made of phosphate buffered saline (PBS) added Tween 80 and a suspension of beads. The beads are used as an internal standard (StdBeads) in the flow cytometer analysis and Tween 80 prevents that beads stick to surfaces. Bead-Mix is prepared in relatively large volumes, distributed e.g. to 50 ml centrifuge tubes and stored frozen for future use. The concentration StdBeads is estimated precisely in a separate analysis (see *Bead-Mix calibration*). Finally, Bead-Mix is the base medium for Stain-Mix (see below).
1. Prepare a solution of PBS (see below)
2. Add 0.5 ml Tween 80 (mat. no. 500479, 0.05% final conc.) per liter of PBS
3. Pass the mixture through a 0.2 µm pore-size filter (e.g. PES 0.2 um 250 ml filtration unit; Nalge-102103-12; VWR)
4. Add 100 ul Yellow-green Fluospheres^{™} (F-8827; Molecular Probes, Leiden, The Netherlands) per liter of PBS (final conc. 0.01% and ca. 4.5E+5 beads/ml)
5. The Bead-Mix preparation is kept mixed to prevent the beads from sinking (e.g. use magnetic stirring)
6. While being stirred, the bead mix is distributed in quantities of 50 g e.g. to 50 ml centrifuge tubes. Liquid may be transferred by an appropriate pipette and the weight of Bead-Mix in the tube is estimated and written on the tube
7. Bead-Mix is stable for at least one week when stored refrigerated. Frozen storage is recommended for longer periods.

### PBS (phosphate buffered saline):

1. 7.6 g NaCl (MW = 58,44; 130 mmol)
2. 0.4 g NaH₂PO₄ · H₂O (MW = 137.99; 3 mmol)
3. 2.5 g Na₂HPO₄ · 12H₂O (MW = 358.14; 7 mmol)
4. Add demineralised water to 1000 g
5. Adjust to pH 6.5 ± 0.1 with NaOH

### Stain-Mix preparation

Stain-Mix is used for the fluorescence staining of bacteria and membrane potential. Prepare fresh the same day of analysis, unless a use-before period is determined. The ingredients are described above and the composition is as follows:
1. 50 g Bead-Mix (see above)
2. Add 105 ul of 1.5 mM DiOC stock solution, if no other instruction is given (3 uM final conc. in the final sample containing a mixture of 24 parts Stain-Mix and 1 part bacteria) 3. Add 210 ul 50% glucose (0.2% final conc. w/w)
4. Mix thoroughly before distribution to Stain-Mix tubes

### FTV-99

FTV-99 abbreviates the Danish word for dilution water (DK: "fortyndingsvand") and denotes a volume of 99 ml. FTV-99 is prepared as follows:
1. 15 g Tryptone (Oxoid L42)
2. 9 g NaCl (Merck no. 106404)
3. 1.14 g Antifoam 1510 (BDH 63215; prepared as 2% stock in demineralised water)
4. Demineralised water to 1000 g
5. pH adjusted to 7.0 ± 0.1 (25°C) using H₃PO₄
6. Autoclaving 121°C for 15 min
7. Stable for 3 months

### MRS

MRS is a general medium to grow Lactobacilli and other lactic acid bacteria. Presently is used MRS-CM from Difco:
1. 55 g MRS - Broth (Difco 288110)
2. 1000 g demineralised water
3. Autoclaving 121°C for 15 min
4. pH = 6.5 ± 0.2 (25°C) after autoclaving

The original MRS recipe by de Man et al. (1960) is:
1. 10 g Oxold peptone
2. 10 g Lab-Lemco (Oxoid)
3. 5 g Yeast extract (Difco or Oxoid)
4. 20 g glucose
5. 1 ml polyoxyethylene sorbitan mono-oleate (Tween 80)
6. 2 g K₂HPO₄
7. 5 g CH₃COONa · 3H₂O
8. 2 g triammonium citrate
9. 0.2 g MgSO₄ · 7H₂O
10. 0.05 g MnSO₄ · 4H₂O
11. 1 liter demineralised water
12. pH 6.0 to 6.5 after autoclaving (120°C, 15 min)

### Sample preparation

Briefly, 1) freeze-dried cell material is rehydrated under agitation for 10 minutes in FTV (0.9% NaCl amended with 1.5% tryptone (Oxoid L42) and 0.1% antifoam 1510 (BDH 63215)) at 25°C. 2) rehydrated cells are then diluted into an appropriate growth medium (in the case of most lactic acid bacteria MRS (de Man et al. 1960) is used. 3) Then cells are incubated in growth medium for 30 min and at the optimal growth temperature of the cells (typically 37 - 40°C in the case of thermophile LAB). This to ensure the cellular energy metabolism is activated, but cell multiplication is minimized. After the activation step,4) the cells are further diluted with Stain-Mix typically, one part activated cells in MRS is mixed with 24 parts stain-mix to obtain a final concentration: 130 mM NaCl, 138 mM NaH2PO4·H2O, 15 mM Na2HPO4·12H2O, 11 mM glucose, 3 uM diethyloxacarbocyanine iodide, 0.05% Tween 80, 0.01% of Yellow-green FluospheresTM (Molecular Probes; Leiden, The Netherlands) and 4% MRS. In certain experiments the MRS concentration of the final mixtures are varied as indicated. Finally 5) the samples are placed at 20-21°C and analysed by FCM.

### Flow cytometry

Flow cytometry is performed using a FacsCalibur (Becton Dickinson) instrument in a standard setup with a 488 nm laser, forward and side scatter detection, and detectors for green, yellow and red fluorescence (all run as logaritmic amplification). The flow medium used is Facsflow (Becton Dickinson).

The read out from the instrument is calibrated by use of the standard beads of the stain-mix by the so-called "Trucount" method.

### EXAMPLE 1: CFU DETERMINATIONS ARE NOT RELATED TO RESULTS OBTAINED WITH VIABILITY TEST BASED ON CELL MEMBRANE INTEGRITY DURING STORAGE.

Lahtinen et al. (2005) found that during 4°C storage of *Bifidobacterium* spp. the counts of colony-forming-units (CFU) decreases, whereas the abundance of cells with intact membrane integrity remain constant using a commercial viability kit (LIVE/DEAD BacLight, Molecular Probes). The authors concluded that Bifidobacteria may enter a dormant state during storage where cells loose their ability to form CFU, but do not necessarily die.

The findings of Lahtinen is verified by work preformed independently at Chr. Hansen A/S by Stavnsbjerg et al. 2003. In brief, freeze-dried *Lactobacillus acidophilus* strain LA-5 was exposed to a three week accelerated storage in air tight aluminium bags for 3 weeks at 30°C (Control: air tight aluminium bags for 3 weeks at -50°C). Cell viability was etimated by determination number of colony forming units (CFU) by the plate count technique, plating on MRS-agar; and incubate at 30°C (see materials and methods section) and quantification of the number of cell cells with intact membrane integrity by flow cytometry. Intact membrane integrity was identified from intracellular accumulation of the dye carboxy-fluorescein and extrusion of the membrane impermeant dye propidium iodide essentially as described by Bunthof et al. (2001) Appl Environ Microbiol. 67, 2326-2335.

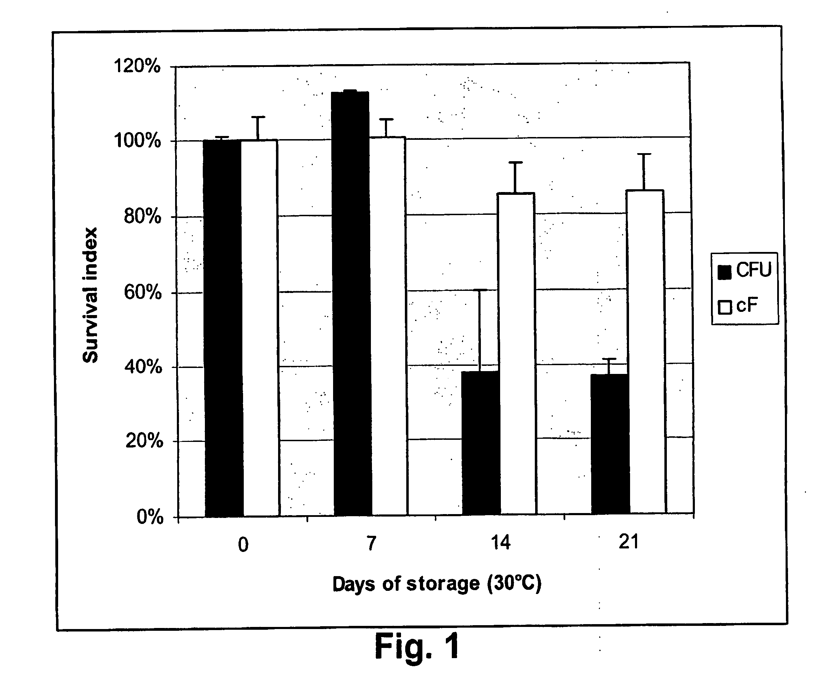

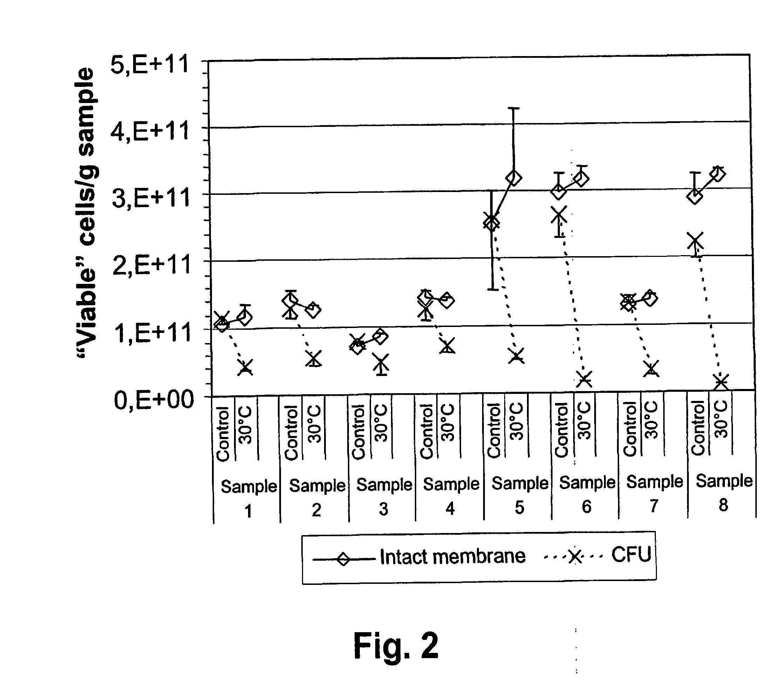

During accelerated storage at 30°C the CFU counts decrease whereas the abundance of cells with intact membrane integrity remains constant, see figure 1 and figure 2. This result is similar to the result reported by Lahtinen et al. (2005) regarding *Bifidobacterium* spp. and shows CFU counts not are related with viability test based on cell membrane integrity during storage of lactic acid bacteria such as *Bifidobacteria* and *Lactobacillus.*

### EXAMPLE 2: THE COMPOSITION OF THE ACTUAL STAINING MIX IS OF MAJOR IMPORTANCE TO THE RESILIENCY OF THE ASSAY

In this experiment the composition of staining mix was studied. The assay was performed essential as described in the materials and methods section. The culture analysed was freeze-dried *Lactobacillus acidophilus* strain LA-5.

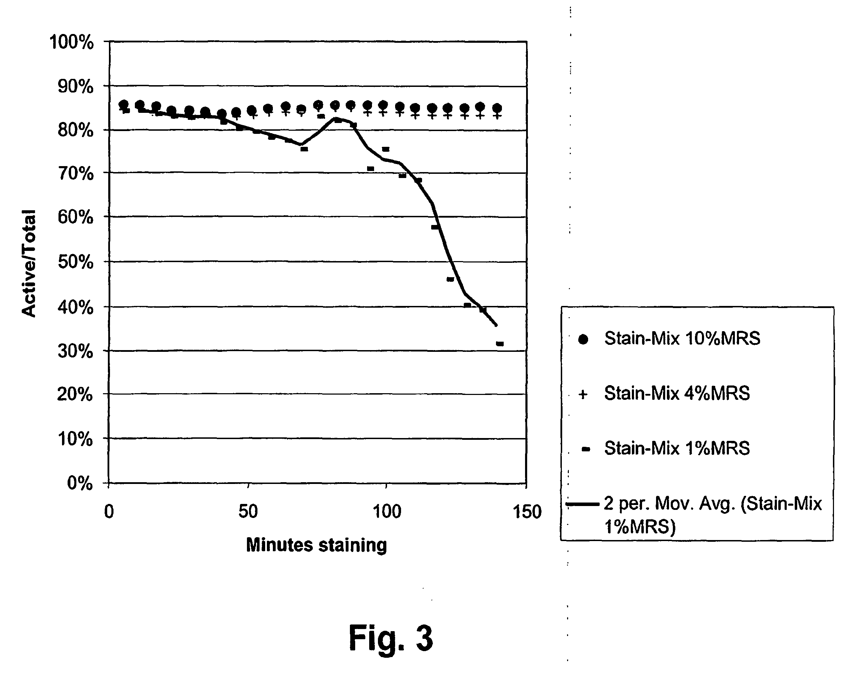

As shown in figure 3 when the stain mix comprising 4 or 10% MRS the viability counts are very resilient resulting in almost identical numbers from 5 to 140 minutes. However stain mix only comprise 1% MRS the viability counts are very dependant on the time in the stain mix. After 140 minutes less than 50% of the number of active cells are detected.

In further experiments it was found that the optimal resilience was obtained when the cell comprising stain mix was kept at temperatures significantly under the optimal growth temperature of the cell.

### EXAMPLE 3: THE VIABILITY DETERMINED BY THE PRESENT PROTOCOL CORRELATES WELL WITH CFU COUNTS.

In this experiment a sample of freeze-dried *Lactobacillus acidophilus* strain LA-5 was analyzed by the membrane potential staining method and compared to the traditional CFU counts. Two applications are exemplified: analysis of standard production and analysis of samples exposed to accelerated storage test.

The direct cell counts of membrane potential expressing cells is done according to the assay described above with duplicate sample analysis. The CFU analysis proceeds as follows: Freeze-dried biomass is rehydrated and diluted in 0.9% NaCl with 1.5% tryptone water, poured to 47°C melted MRS-agar and incubated 3 days at 37°C until counting of CFU. Two to six independent replicates were done.

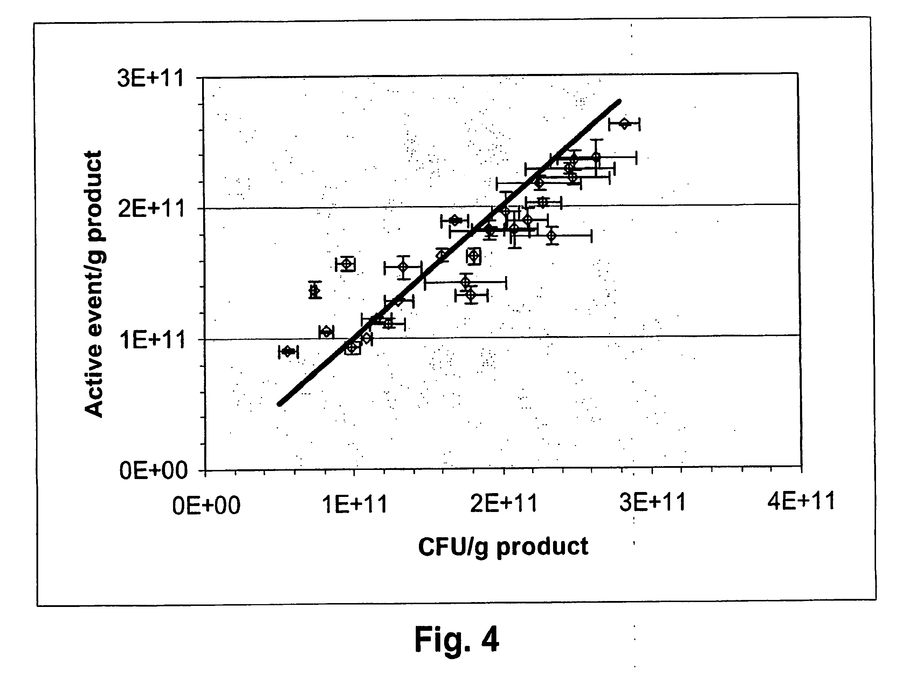

For a standard productions of *Lactobacillus acidophilus* strain LA-5 there is a significant positive correlation between the two methods (r2 = 0,79; P < 0.05) and the bias is within a factor of two (figure 4).

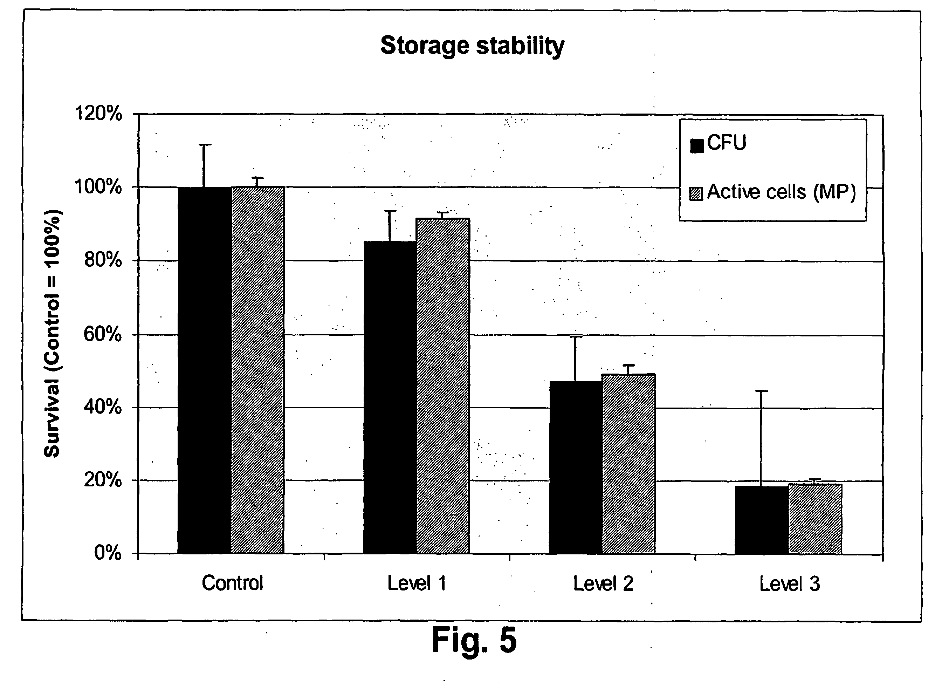

Accelerated storage stability were tested by 3 weeks storage at 30°C in air tight aluminium bags (Level 1; equivalent to "30°C" in Figure 2) and in open air at relative humidities of either 15% (Level 2) or 30% (Level 3). Control samples are stored at -50°C for 3 weeks.
As can be seen from figure 5 the number of viable cells quantified by CFU counting and the membrane potential method of the present invention correlates excellently.

### EXAMPLE 4: VERIFICATION OF ROBUSTNESS IN CELL ACTIVATION - REPEATED ANALYSES.

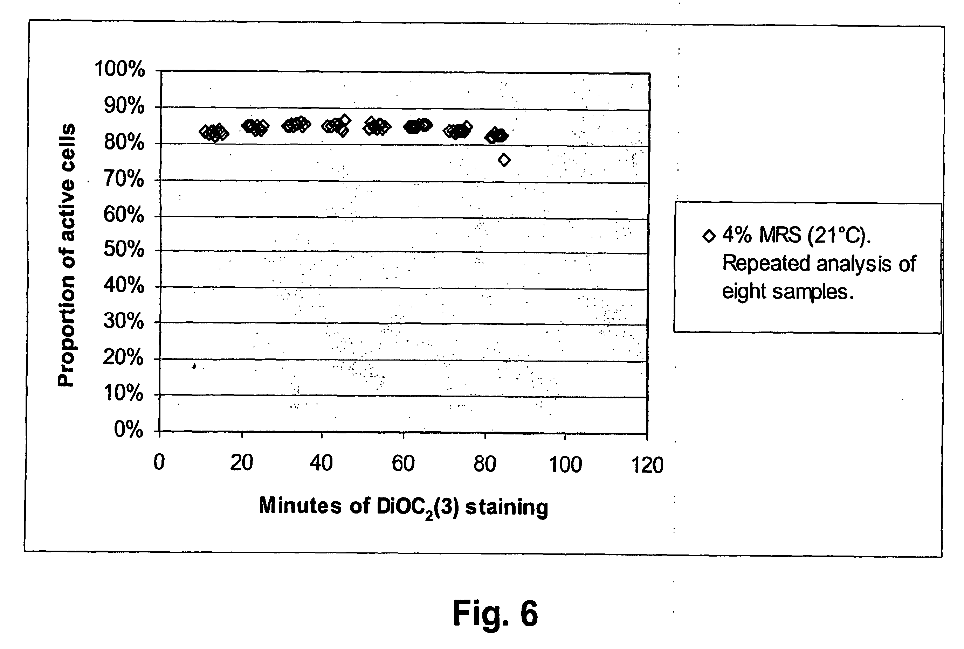

The method is very robust, which provides a basis for very precise measurements multiple samples. In this experiment the reproducibility of the assay is demonstrated by comparing the results from nine individual experiments each of which comprising eight different samples. The eight samples are stained with DiOC₂(3) for intervals between 10-15 minutes and 80 minutes. The multiple measurements average 84% active cells with a standard deviation of 1.5%. The very low standard deviation for the analysis of differently treated samples is evidence that the cell activation is stabilized by the incubation conditions incl. 4% MRS at 20°C. The result is indicated in figure 6 and clearly shows a very high degree of reproducability.

### EXAMPLE 5: EXAMPLE: VERIFICATION OF ROBUSTNESS IN CELL ACTIVATION - ASSAY LINEARITY.

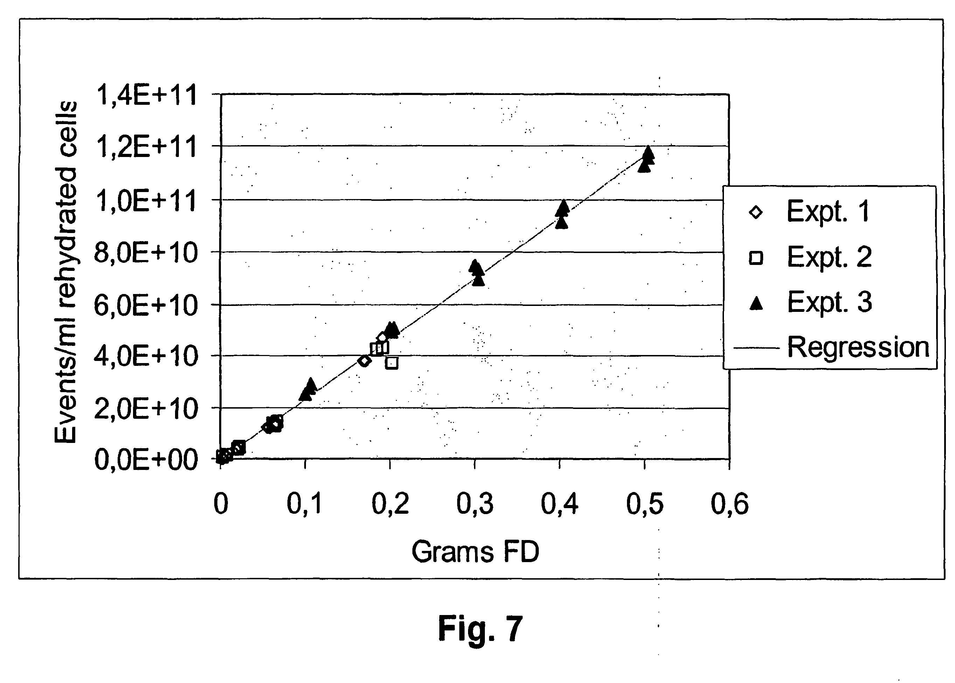

Another example of the precise counts of active cells by the present invention is provided by an analysis of the dose-response relationship between amount of sample and counts of active cells, as shown in the figure below. Three independent experiments were performed (Expt. 1-3) and the regression coefficient is r2 = 0.996 over the entire range clearly indicating that the method is resilient and can be used on samples comprising a wide range of living cells - see figure 7.

### REFERENCES

Lahtinen SJ, M Gueimonde, AC Ouwehand, JP Reinikainen, SJ Salminen: Probiotic Bacteria May Become Dormant during Storage. Appl Environ Microblol, 71(3): 1662-1663 (2005)
de Man JC, Rogosa M, Sharpe ME (1960): A medium for the cultivation of Lactobacilli. J Appl. Bacteriol 23: 130-135
Shapiro, ASM news 56, 584-588 (1990).
Shapiro & Nebe-von-Caron (2004) Multiparameter flow cytometry of bacteria. In: Methods in Molecular Biology: Flow cytometry protocols (2nd edition). Editor: TS Hawley, RG Hawley. Humana Press Inc., Totowa, NJ
Stavnsbjerg R, Knap I, Worm J (2003). Flow cytometric assessment of membrane damage during freezing, freeze-drying and storage of lactobacillus acidophilus. Poster at conference: CryoBiomol 2003, Coimbra 14 - 18 September 2003.
Bunthof et al.: Flow Cytometric Assesment of Viability of Lactic Acid Bacteria. Appl Environ Microbiol. 67, 2326-2335 (2001).
Novo et al. Cytometry 35:55-63 (1999)
Sims et al.: Biochemistry 13: 3315-3330 (1974)
P.K. Horan and L.L. Wheeless, Jr. in Science, Vol. 198, pages 149-157 (1977)
Ben Amor et al. : Appl. Environ Microbiol. 68,5209-5216 (2002)

## Claims

1. A method of quantifying the number of viable lactic acid bacteria (LAB) cells in a sample comprising the steps of:
1: preparing a composition comprising:
a) the LAB cells;
b) a dye which associates with individual LAB cells and which is indicative of the cell membrane potential of said individual cells;
c) a complex growth medium in a concentration that is 2% to 20% (calculated of the composition as a whole) of the recommended concentration when used as a growth medium for LABs.;
2: incubating under conditions where viable cells retain said cell membrane potential while their growth is inhibited;
3: detecting an optical property of the dye composition associated with said individual cells; and
4: quantifying the number of viable cells in the sample.

2. The method according to claim 1, wherein the complex growth medium is MRS.

3. The method according to any of the preceding claims, wherein the incubation in step (2) is performed at a temperature, which is at least 10°C lower than the optimal growth temperature of the lactic acid bacteria cells.

4. The method according to claim 3, wherein the lactic acid bacteria are mesophilic organisms having optimum growth temperatures at about 30°C.

5. The method according to claim 3, wherein the lactic acid bacteria are thermophilic organisms having optimum growth temperatures in the range of 40 to 45°C.

6. The method according to claim 4, wherein the incubation in step (2) is performed at a temperature in the range between 10°C to 25°C.

7. The method according to any of the preceding claims, wherein the detection in step (3) is performed by flow cytometry, e.g. by measuring fluorescence.

8. The method according to any of the preceding claims, wherein the dye composition comprises glucose at a concentration which is 0,5-10%, preferably 1 - 10% and most preferred 2-5% vol/vol (final concentration).

9. The method according to any of the preceding claims, wherein the dye is DiOC.

## Patentansprüche

1. Verfahren zum Quantifizieren der Zahl der lebensfähigen Milchsäurebakterien(LAB)-Zellen in einer Probe, mit folgenden Phasen:
1: Herstellung einer Zusammensetzung mit:
a) den Milchsäurebakterienzellen;
b) einer Farbe, die mit individuellen Milchsäurebakterienzellen assoziiert und die das Zellmembranpotential der individuellen Zellen anzeigt;
c) einem komplexen Wachstumsmedium in einer Konzentration von 2% bis 20% (der gesamten Zusammensetzung) der empfohlenen Konzentration bei Verwendung als Wachstumsmedium für Laboratorien;
2: Inkubierung unter Bedingungen, bei denen lebensfähige Zellen besagtes Zellmembranpotential behalten, während ihr Wachstum gehemmt wird;
3: Erfassung einer optischen Eigenschaft der Farbenzusammensetzung, die mit besagten individuellen Zellen assoziiert ist, und
4: Quantifizierung der Zahl der lebensfähigen Zellen in der Probe.

2. Verfahren nach Anspruch 1, wobei das komplexe Wachstumsmedium MRS ist.

3. Verfahren nach einem beliebigen der vorherigen Patentansprüche, wobei die Inkubation in Phase (2) bei einer Temperatur durchgeführt wird, die mindestens 10 °C niedriger als die optimale Wachstumstemperatur der Milchsäurebakterienzellen ist.

4. Verfahren nach Anspruch 3, wobei die Milchsäurebakterien mesophile Organismen mit optimalen Wachstumstemperaturen von etwa 30°C sind.

5. Verfahren nach Anspruch 3, wobei die Milchsäurebakterien wärmeliebende Organismen mit optimalen Wachstumstemperaturen im Bereich von 40 bis 45°C sind.

6. Verfahren nach Anspruch 4, wobei die Inkubation in Phase (2) bei einer Temperatur in dem Bereich zwischen 10°C und 25°C durchgeführt wird.

7. Verfahren nach einem beliebigen der vorherigen Patentansprüche, wobei die Erkennung in Phase (3) mittels Durchflusszytometrie durchgeführt wird, z.B. durch Messung der Fluoreszenz.

8. Verfahren nach einem beliebigen der vorherigen Patentansprüche, wobei die Farbenzusammensetzung Traubenzucker in einer Konzentration von 0,5-10% umfasst, vorzugsweise 1 - 10% und besonders 2-5% vol/vol (Endkonzentration).

9. Verfahren nach einem beliebigen der vorherigen Patentansprüche, wobei die Farbe DiOC ist.

## Revendications

1. Procédé pour quantifier le nombre de cellules de bactéries d'acide lactiques (BAL) viables dans un échantillon, comprenant les phases de :
1: préparation d'une composition comprenant :
a) les cellules BAL;
b) un colorant qui s'associe avec des cellules BAL individuelles et qui est indicatif du potentiel de membrane de cellule desdites cellules individuelles;
c) un milieu de croissance complexe dans une concentration allant de 2% à 20% (calculé selon la composition dans son ensemble) de la concentration conseillée quand celle-ci est utilisée comme milieu de croissance pour les BAL;
2: incubation dans des conditions où les cellules viables conservent ledit potentiel de membrane de cellule tandis que leur croissance est inhibée;
3: détection d'une propriété optique de la composition de colorant associée avec lesdites cellules individuelles; et
4: quantification du nombre de cellules viables dans l'échantillon.

2. Procédé selon la revendication 1, où le milieu de croissance complexe est MRS.

3. Procédé selon l'une quelconque des revendications précédentes, où l'incubation dans la phase (2) est accomplie à une température qui est au moins de 10 °C inférieure à la température de croissance optimale des cellules de bactéries d'acide lactique.

4. Procédé selon la revendication 3, où les bactéries d'acide lactique sont des organismes mésophiles ayant des températures de croissance optimales à environ 30°C.

5. Procédé selon la revendication 3, où les bactéries d'acide lactique sont des organismes thermophiles ayant des températures de croissance optimales de l'ordre de 40 à 45°C.

6. Procédé selon la revendication 4, où l'incubation dans la phase (2) est accomplie à une température entre 10°C et 25°C.

7. Procédé selon l'une quelconque des revendications précédentes, où la détection dans la phase (3) est accomplie par la cytométrie en flux, p. ex. en mesurant la fluorescence.

8. Procédé selon l'une quelconque des revendications précédentes, où la composition de colorant comprend du glucose dans une concentration allant de 0,5 à 10%, de préférence 1 - 10% et mieux encore de 2 - 5% vol/vol (concentration finale).

9. Procédé selon l'une quelconque des revendications précédentes, où le colorant est DiOC.
